# EUROPÄISCHE PATENTSCHRIFT

(11) **EP 0 626 974 B1**
(45) Veröffentlichungstag und Bekanntmachung des Hinweises auf die Patenterteilung: **19.03.1997**
(21) Anmeldenummer: 93903800.6
(22) Anmeldetag: 12.02.1993
(51) Int. Cl.: C08B 37/04, A61L 31/00, A23L 1/0532, A61L 15/00, A61K 6/10, A61K 9/48, A61K 47/36, A61K 49/00

(54) **MITOGENFREIE SUBSTANZ, DEREN HERSTELLUNG SOWIE VERWENDUNG**
MITOGEN-FREE SUBSTANCE, ITS PREPARATION AND ITS USE
SUBSTANCE EXEMPTE DE MITOGENE, SA FABRICATION ET SON APPLICATION

(30) Priorität: 12.02.1992 DE 4204012
(43) Veröffentlichungstag der Anmeldung: 07.12.1994
(73) Patentinhaber: MONSANTO EUROPE S.A., B-1150 Brussels (BE)
(72) Erfinder: ZIMMERMANN, Ulrich, D-8702 Waldbrunn (DE); FEDERLIN, Konrad, D-6300 Giessen (DE); ZEKORN, Tobias, D-6300 Giessen (DE); KLÖCK, Gerd, D-8702 Zell am Main (DE)
(74) Vertreter: Bosch, Henry
(86) Internationale Anmeldenummer: DE9300136
(87) Internationale Veröffentlichungsnummer: WO9316111

(56) Entgegenhaltungen:
- ELECTROPHORESIS Bd. 13, Nr. 5, 1992, WEINHEIM, DE Seiten 269 - 274 U. ZIMMERMANN ET AL. 'Production of mitogen-contamination free alginates with variable ratios of mannuronic acid to guluronic acid by free flow electrophoresis'
- JOURNAL OF CHROMATOGRAPHY Bd. 89, 1974, IT Seiten 99 - 102 C. BUCKE 'Polyacrylamide gel electrophoresis of alginic acid'
- DATABASE WPIL Section Ch, Week 8644, Derwent Publications Ltd., London, GB; Class D, AN 56-290660 & SU,A, 1 219 041

## Beschreibung

Die Erfindung betrifft eine mitogenfreie Substanz unter Verwendung von Guluron-Säure oder MannuronSäure, deren Herstellung sowie Verwendung.

Gemäß üblicher Terminologie definiert der Begriff mitogen jene Substanzen, die Zellen zu Teilungen anregen, die andernfalls, d.h. ohne Einwirkung dieser Substanz, nicht zu einer Teilung geführt würden. Die hierdurch im Organismus ausgelösten Reaktionen sind von der Norm abweichend und können sich als allergische, immunologische und pyrogene (Entzündungs-) Reaktionen äußern. Keiner näheren Erläuterungen bedarf, daß die Schaffung und Herstellung mitogenfreier Substanzen für den Kontakt mit lebenden Organismen in Biologie und Medizin von allergrößtem Interesse sind.
Auf dem Gebiet der Implantatchirurgie ist der Einsatz mitogenfreier Substanzen von allergrößter Bedeutung, um die anderenfalls induzierten und zur Abstoßung des Implantats führenden immunologischen Reaktionen zu unterdrücken. Bei zahlreichen Erkrankungen werden lebende Zellen in den Organismus eingebracht, die einerseits durch Nährstoffe versorgt werden und andererseits entsprechend dem jeweiligen Bedarf Zellsekrete, Hormone oder dgl. produzieren und abgeben, um eine Unterproduktion oder den völligen Ausfall der körpereigenen Zellen zu kompensieren. Eine der bekanntesten Erkrankungen dieser Art ist die Diabetes, bei der zwar die Fehlmenge des benötigten Insulins im zeitlichen Abstand durch wohldosierte äußere Zufuhr ausgeglichen werden kann, bei der jedoch festzustellen ist, daß eine exakte Dosierung über den Tagesverlauf im Sinne eines Regelsystemes nie realisierbar ist, so daß Schwankungen und Abweichungen vom Idealwert unvermeidbar sind. Dieser Nachteil läßt sich durch Implantieren lebender Zellen in den Organismus beseitigen, wobei jedoch zur Unterdrückung immunologischer Reaktionen die Zellen in Kapseln unterzubringen sind, die ihrerseits folgenden Anforderungen genügen müssen: Zum einen muß eine ungehinderte Diffusion durch die Wandungen im Sinne einer Zufuhr von Nährstoffen und andererseits die Abgabe der Zellsekrete, Hormone und dgl. nach außen gewährleistet sein. Weiter muß sichergestellt sein, daß das Implantat keine Immunreaktionen hervorruft, d.h. mitogenfrei ist, was sich u.a. anderenfalls dadurch äußern würde, daß sich die Kapsel oberflächlich nach einer gewissen Dauer mit fibrotischem Bewuchs zusetzen und die unabdingbare Zirkulation und Austausch von Nährstoffen und Zellsekreten unterbinden würde.

Ziel vorliegender Erfindung ist zunächst die Schaffung einer mitogenfreien Substanz, die auch nach längerem Kontakt mit dem lebenden Organismus keine allergischen, immunologischen und pyrogenen Reaktionen zeigt sowie deren Herstellung und Verwendung.

Gelöst wird diese Aufgabe erfindungsgemäß durch Mischpolymere, die aus 10 bis 90 Molprozent Guloronsäure und aus jeweils auf 100 % ergänzend Mannuronsäure bestehen sowie ein Molekulargewicht der Mischpolymere von 10 000 bis 500 000 Dalton.

Die mitogenfreie Substanz weist als unter Umständen einzige Komponente ein Mischpolymer aus Guluron-säure und Mannuronsäure, wobei die molare Zusammensetzung der Guluronsäure 10 bis 90 Prozent und der Rest Mannuronsäure beträgt. Eine Aussage über die Größe des sich durch Polymerisation ausbildenden Mischpolymers ist aufgrund des vorgegebenen Molverhältnisses beider Säuren noch nicht festgelegt. Es können demnach auch Mischpolymere mit niedrigem Molekulargewicht beschrieben werden, die die gestellten Anforderungen nicht erfüllen. Zusätzliche Voraussetzung ist deshalb, daß das Mischpolymer ein Molekulargewicht von 10 000 bis 500 000 Dalton besitzt. Diese Molekulargewichte lassen sich relativ einfach durch Verwendung von Membranen und Filter mit geeigneter Durchlässigkeit und Porenradius durch Dialyse gewinnen.

Der Nachweis der Mitogenfreiheit geschieht in der Weise, daß die mit Calcium polymerisierte Substanz auf das Induzieren in vitro von meßbarer Proliferation von Milz-Lymphozyten überprüft wird. Zu diesem Zwecke werden Milzzellen aus Mäusen des Inzuchtstammes Balb-c gewonnen. Die Zellen wurden bei einer Zellzahl von 1·10⁶ ml⁻¹ in Wachstumsmedium RPMI 1640 Medium, 10% fötales Kälberserum (Boehringer, Mannheim, Deutschland), 2 mM L-Glutamin, 2 mM Natriumpyruvat, nichtessentieller Aminosäure (1x, Boehringer, Mannheim, Deutschland), 50 µM 2-Mercaptoethanol, 100 units ml⁻¹ Penicillin und 100 µg ml⁻ ¹ Streptomycin (Biochrom, Berlin, Deutschland) in Gegenwart von 100 µg ml⁻¹ der mitogenen Substanz bei 37° Celsius kultiviert. Die Lymphozyten zeigten weder einen signifikanten Einbau von ¹⁴C- oder ³H-Thymidin in säurefällbare zelluläre Substanzen (nach 3 Tagen gemessen) noch ein mikroskopisch feststellbares Wachstum (mikroskopische Zellzahlbestimmung nach 5 bis 9 Kulturtagen).

Im Rahmen der Erfindung steht grundsätzlich frei, ob neben dem vorgenannten Mischpolymer zusätzliche, im Hinblick auf den Mitogenitätstest ebenfalls unbedenkliche Komponenten beigemischt sind.

Aus der Mitogenfreiheit der Substanz folgt nicht zwingend die Biokompatibilität in vivo. Vielmehr hängt letztere Eigenschaft von weiteren Faktoren, u.a. der Oberflächenbeschaffenheit des Implantats sowie dem Implantationsort ab. Dennoch ist von Vorteil die sich als biokompatibel erweisenden Verbindungen auszuwählen. Die Biokompatibilität wird anhand von Tierversuchen an Mäusen und Ratten im Hinblick auf das Auslösen von Fremdkörperreaktionen untersucht. In konkreter Durchführung werden hierzu Kapseln aus mit Barium oder Kalzium polymerisierten Substanzen hergestellt und intraperitoneal in diesen Tieren implantiert. Es trat weder ein fibrotischer Bewuchs noch eine Fremdkörperreaktion im umgebenden Gewebe auf. Im konkreten Fall wurde eine zweiprozentige Lösung der Substanz in 0,9 % NaCl mit 20 mM BaCl₂ polymerisiert, wodurch sich Gelkugeln von 0,5-1 mm Durchmesser bildeten. Diese wurden mit 0,9 % NaCl gewaschen, zwei Tage lang in RPMI 1640 Medium inkubiert und dann in das Peritoneum von Mäusen und Ratten implantiert. Nach drei Wochen wurden die Kapseln durch Ausspülen des Peritoneums zurückgewonnen und histologisch analysiert.

Eine weitere zusätzliche Charakterisierung der mitogenfreien Substanz macht sich die elektrophoretische Beweglichkeit zunutze und ist wie folgt: Die Substanz besteht aus einer als Mischpolymer ausgebildeten Komponenten, die aus 10 bis 90 Molprozenten Guluronsäure und komplementär, d.h. 90 bis 10 % Mannuronsäure, aufgebaut ist. Die aufgrund dieser chemischen Zusammensetzung definierten und möglichen Mischpolymere können im Hinblick auf die Molekülgröße und der jeweiligen molekularen Konfiguration sehr unterschiedlich sein. Um hieraus die mitogenfreie Substanz zu gewinnen, werden die unterschiedlichen Mischpolymere einer trägerfreien Elektrophorese unterzogen, wobei die hier interessierenden Fraktionen einer Beweglichkeit zwischen 3-5 · 10⁻⁴ cm² · V⁻¹ Ø s⁻¹ aufweisen.
Spezielle Versuche in diesem Zusammenhang wurden bei einer Trennkammerdimension von 30 · 130 · 0,3 mm, einer Kammerpuffertemperatur von 22° C, einer Kammerpufferzusammensetzung von 15 mM Triethanolamin, 7,1 mM Kaliumazetat, 216 mM Glycin (pH 7,2), 11 mM Glukose, 0,2 mM Ethylendiaminotetraacetat, 100 Einheiten ml⁻¹ Penicillin, 100 µg · ml⁻¹ Streptomycin, Leitfähigkeit 1,9 mS cm⁻¹. Der Puffer wurde mit einer Geschwindigkeit von 2,5 ml · h⁻¹ durch die Kammer gepumpt. Die Pumpe wurde mit einer Rate von 350 µl · h⁻¹ injiziert. Die Feldstärke betrug 100 V · cm⁻¹ (die Stromstärke 65 mA). Als zusätzliche Bedingung muß die Substanz ein Molekulargewicht zwischen 10 000 und 500 000 Dalton aufweisen. Im Wege der Diffusion durch eine Membran lassen sich eine Separation der Mischpolymere dieses Molekulargewichtes erreichen.

Die Bedeutung der erfinderischen Leistung der hier vorgeschlagenen Substanz läßt sich daraus erkennen, daß die Fachwelt unter Hinweis auf konkrete Erfahrungen und Versuche der Auffassung ist, daß Mannuronsäure und manuronsäurehaltige Mischpolymere als Substanz selbst mitogen wäre (Fundstelle Journal of Immunotherapy 10, Seite 286-291, 1991 Raven Press, New York; Transplantation Proceedings Vol. 23, No. 1 (February) 1991, Seite 758-759). Das Verdienst vorliegender Erfindung ist die Erkenntnis der Unrichtigkeit dieser Behauptung. Hinzu kommt weiter, daß Alginate grundsätzlich mitogene Reaktionen zeigen, die jedoch - hierin liegt eine weitere entscheidende Erkenntnis - durch die aufgrund der Herleitung und Extraktion aus Naturprodukten nicht auszuschließenden Verunreinigung durch Umwelteinflüsse zurückzuführen sind.

Bei entsprechenden Herstellungsverfahren lassen sich ausgehend Rohalginaten Substanzen herleiten, die sich durch mitogenfreie Eigenschaften auszeichnen.

Im folgenden werden zwei Verfahren zur Herstellung der vorerwähnten mitogenfreien Substanzen angegeben. Im ersteren finden Verfahren der trägerfreien Elektrophorese Anwendung und im zweiten erfolgt eine Gewinnung durch chemische Reaktion. Beiden Verfahren gemeinsam ist das Ausgangsmaterial Alginat, was durch Extraktion aus Pflanzen, Algen, Bakterien und dgl. gewonnen wird und käuflich erwerbbar ist.
Eine bis zu 10%-ige Alginatlösung wird in einen Elektrophoresepuffer mit einer elektrischen Leitfähigkeit von 2 mS · cm⁻¹ eingegeben. Es erfolgt eine Trennung in einzelne Fraktionen unterschiedlicher elektrophoretischer Beweglichkeit, die getrennt aufgefangen werden. Es interessiert hier eine elektrophoretische Beweglichkeit von 3-5 · 10⁻⁴ cm² · V⁻¹ · s⁻¹· Anschließend erfolgt in einer zusätzlichen Dialyse gegen Wasser eine Separation nach der Diffusionsfähigkeit, die sich durch das Molekulargewicht unterscheidet. Zur Erhaltung einer mitogenfreien Substanz werden Molekulargewichte von 10 000 bis 500 000 Dalton aus dem gesamten Mischpolymer gewonnen.

Das nunmehr im folgenden beschriebene Herstellungsverfahren auf chemischer Basis ist dadurch charakterisiert, daß das durch Extraktion aus Pflanzen, Algen, Bakterien oder dgl. gewonnene Alginat zunächst zu einem unlöslichen Komplex vernetzt, der eine Reinigung und Extraktion sowie eine anschließende Rückgewinnung zuläßt. Bei diesem Verfahren werden die Alginate durch Zugabe von Ba⁺⁺ -Ionen oder vergleichbaren multivalenten Kationen (Blei Pb, Kupfer Cu), die eine vergleichbare oder höhere Affinität zum Alginat haben als Barium, ausgefällt. Anschließend erfolgt eine Extraktion durch Säure, wie beispielsweise Essigsäure über mehrere Stunden bei erhöhter Temperatur. Nach einem Waschen folgen weitere Extraktionen mit Komplexbildnern, wie z.B. Zitronensäure im alkalischen Bereich. Nach Waschen im destillierten Wasser werden die Gelkugeln mehrere Stunden mit Alkohol, z.B. Äthanol, gerührt. Das Freisetzen der nunmehr gereinigten, jedoch nach wie vor vernetzten Alginate erfolgt durch Beigabe von EDTA (Ethylendiamin-Tetraessigsäure) im stark alkalischen Bereich. Hierbei lösen sich die Gelkugeln auf. Das Resultat ist eine aus Rohalginat gewonnene mitogenfreie Substanz.

Im folgenden wird ein auf chemischer Basis durchgeführter Herstellungsvorgang in allen Einzelheiten beschrieben:

Eine 2 bis 4%-ige Lösung von Rohalginat wird über ein Membranfilter von 0,22 µm Porenweite filtriert und dann unter Rühren in das 5-fache Volumen einer 50 mM-Bariumchloridlösung eingetropft, wodurch Gelkugeln von ca. 1-5 mm Durchmesser entstehen. Nach 10 bis 20 Minuten erfolgt ein Waschen der Gelkugeln in destilliertem Wasser und anschließend zweimal für 3-4 Stunden in je 1 Liter 1M Essigsäure/10 mM BaCl₂ bei 60-80°C gerührt. Anschließend werden die Gelkugeln auf einem Sieb mit destilliertem Wasser gewaschen und über Nacht in zweimal 1 Liter 500 mM Zitronensäure (pH-Wert 8,0 mit KOH) gerührt, wobei nach 4 Stunden ein Mediumwechsel erfolgt. Nach nochmaligem Waschen der Gelkugeln im destillierten Wasser und für 2 - 3 Stunden in 500 ml 80 % Ethanol werden sie anschließend für je 1 Stunde in 1 bis 2 Stunden in destilliertem Wasser gerührt.
Die Gelkugeln werden über ein Sieb dekantiert und in 200 ml einer 250 mM EDTA-Lösung (pH 10,0 mit KOH) suspendiert. Nach Rühren über Nacht haben sich die Kugeln aufgelöst. Die Lösung wird dreimal für mindestens 4 Stunden gegen 2-3 Liter destilliertem Wasser (pH 8,5 mit KOH) dialysiert und dann gefriergetrocknet.

Ohne Einschränkung der Allgemeinheit werden im folgenden einige Anwendungen der erfindungsgemäß angegebenen mitogenfreien Substanzen aufgezeigt. Sie haben gemeinsam, daß sie vom tierischen oder vom menschlichen Körper problemlos aufgenommen und auch bei langzeitlicher Anwendung die Auslösung mitogener Reaktionen mit Sicherheit ausschließen.

Als erstes ist das Gebiet der Transplantationschirurgie zu erwähnen, bei welchem mitogenfreie Substanzen dazu eingesetzt werden können, lebende Zellen nach Art einer Kapsel einzuschließen und ohne die Auslösung immunologischer Reaktionen im Körper des Menschen implantieren zu können. Als besonders bedeutungsvolles Beispiel wird die Einbringung von insulinerzeugenden Zellen (Inseln) in die aus mitogenfreien Substanzen bestehende Kapsel angegeben, die ausweislich von Tierversuchen auch nach längerer Implantation nicht zu fibrotischem Bewuchs neigen. Gleichzeitig erlaubt die Kapsel das Eindringen der für die Aufrechterhaltung des Zellwachstums erforderlichen Nährstoffe und andererseits die Abgabe der hierbei erzeugten Hormone - im Falle der Diabetes handelt es sich um das Insulin - nach außen zu ermöglichen. Man macht sich die Durchlässigkeit für Glucose, Sauerstoff, Peptid-Hormone, zu denen das Insulin zu rechnen ist, zunutze, bei gleichzeitiger Undurchlässigkeit für größere Moleküle, z.B. der Antikörper. Das Anbringen lebender Zellen (Langerhaussche Inseln) erlauben eine ideale Anpassung im Sinne eines Regelprozesses an den augenblicklichen Hormonbedarf.

Weiterhin eignet sich die Verwendung mitogenfreier Substanzen in besonderer Weise zur Beigabe zu Nahrungsmitteln, wo sie bekanntlich die Gelierung und Stabilisierung unterstützen. Statistische Untersuchungen lassen Zusammenhänge zwischen der Verwendung der (bisherigen) Alginate mit der Häufigkeit der Entstehung von Krebs des Magen/Darmtraktes vermuten. Diese momentan eingesetzten Alginate sind mit Verunreinigungen versehen und deshalb, wie die Erfindung erkannt hat, mitogen. Der Ersatz durch mitogenfreie Alginate hätte erhebliche gesundheitliche Vorzüge.

Schließlich lassen sich mitogenfreie Substanzen als Wundverschlüsse bei Verletzungen, als auch innerlich bei Geschwüren, insbesondere Magengeschwüren, einsetzen. Sie können als zahnärztliche Abdruckmassen gefahrlos Verwendung finden und schließlich in der Galenik als Kapseln oder Kugeln, die als Trägersubstanz für Medikamente dienen und die sich im Magen oder Darm unter Freisetzung des eingeschlossenen Medikamentes auflösen. Gleichermaßen kann es als Trägersubstanz für Kontrastmittel dienen, in dem z.B. Luft in Bläschenform aufgenommen und eingeschlossen wird, das der Kontrastierung bei Ultraschallaufnahmen dient.

## Patentansprüche

1. Mitogenfreie Substanz unter Verwendung von Guluron-Säure oder Mannuron-Säure, **gekennzeichnet durch** Mischpolymere, die aus 10 bis 90 Molprozent Guluronsäure und aus jeweils auf 100 % ergänzend Mannuronsäure bestehen sowie ein Molekulargewicht der Mischpolymere von 10 000 bis 500 000 Dalton aufweisen.

2. Substanz nach Anspruch 1, **gekennzeichnet durch** Biokompatibilität.

3. Mitogenfreie Substanz nach Anspruch 1 **gekennzeichnet durch** eine elektrophoretische Beweglichkeit der Mischpolymere von 3-5 · 10⁻⁴ cm² · V⁻¹ · s⁻¹.

4. Verfahren zur Herstellung der mitogenfreien Substanz nach einem der Ansprüche 1 bis 3, **dadurch gekennzeichnet,** daß eine bis zu 10%-ige Alginatlösung durch trägerfreie Elektrophorese in unterschiedliche Fraktionen separiert wird und anschließend die Fraktion mit einer elektrophoretischen Beweglichkeit von 3-5 · 10⁻⁴ cm² · V⁻¹ · s⁻¹ eine Dialyse zur Separierung der Moleküle mit einem Gewicht von 10 000 bis 500 000 Dalton erfolgt.

5. Verfahren zur Herstellung einer mitogenfreien Substanz nach einem der Ansprüche 1 bis 3, **dadurch gekennzeichnet,** daß die Alginatlösung durch die Zugabe von Barium oder multivalenten Kationen (z.B. Blei Pb, Kupfer Cu), die eine vergleichbare oder höhere Affinität zum Alginat als Barium haben, zu einem unlöslichen Komplex ausgefällt werden, dann eine Extraktion durch Säure, z.B. Essigsäure, über mehrere Stunden bei hoher Temperatur erfolgt, anschließend nach einer Waschung eine weitere Extraktion mit Komplexbildner, z.B. Zitronensäure, im alkalischen Bereich über mehrere Stunden vorgenommen wird, nach einem weiteren Waschen mit destillierten Wasser eine Behandlung über mehrere Stunden mit Alkohol, z.B. Ethanol, und schließlich ein Freisetzen der Alginate durch Zugabe von EDTA (Ethylendiamin-tetraessigsäure) im stark-alkalischen Bereich erfolgt.

6. Mitogenfreie Substanz nach einem der Ansprüche 1 bis 3, **dadurch gekennzeichnet,** daß sie als Implantat, vorzugsweise als im Innern mit lebenden Zellen bestückte Kapseln, in der Transplantationschirurgie verwendet werden.

7. Mitogenfreie Substanz nach einem der Ansprüche 1 bis 3, **gekennzeichnet** als Stabilisierungs- und Gelierzusatz für Nahrungsmitteln.

8. Mitogenfreie Substanz nach einem der Ansprüche 1 bis 3, **gekennzeichnet** als innerlich und/oder äußerlich angewandtes Wundverschlußmaterial und/oder zahnärztliche Abdruckmasse und/oder für als Trägersubstanz für Medikamente oder Kontrastmittel dienende Kapseln und Kugeln.

## Claims

1. Mitogen-free substance using guluronic acid or mannuronic acid, characterised by copolymers consisting of 10 to 90 mol% of guluronic acid and, supplementing each to 100%, of mannuronic acid, and the copolymers having a molecular weight of 10,000 to 500,000 dalton.

2. The substance in accordance with Claim 1, characterised by biocompatibility.

3. The mitogen-free substance in accordance with Claim 1, characterised by an electrophoretic mobility of the copolymers of 3 to 5 . 10⁻⁴ cm². V⁻¹ . s⁻¹.

4. A process for the preparation of the mitogen-free substance in accordance with one of Claims 1 to 3, characterised in that an up to 10% alginate solution is separated into different fractions by carrier-free electrophoresis and subsequently, the fraction with an electrophoretic mobility of 3 to 5 . 10⁻⁴ cm² . V⁻¹ . s⁻¹ is dialyzed in order to separate molecules with a weight of 10,000 to 500,000 dalton.

5. A process for the preparation of a mitogen-free substance in accordance with one of Claims 1-3 characterised in that the alginate solution is precipitated into an insoluble complex by adding barium or multivalent cations (e.g. lead Pb, copper Cu), which have a similar or higher affinity for the alginate than barium; followed by an extraction with an acid, such as acetic acid, for several hours at increased temperatures; after washing, another extraction is conducted over several hours in an alkaline range using a complexing agent, such as citric acid; after further washing in distilled water, a treatment with an alcohol, such as ethanol, is conducted over several hours and finally the alginates are released by addition of EDTA (ethylenediamine tetraacetic acid) in a highly alkaline range.

6. Mitogen-free substance in accordance with one of Claims 1 to 3, characterised in that it is used as an implant, preferably in the form of capsules containing living cells, in transplant surgery.

7. Mitogen-free substance in accordance with one of Claims 1 to 3, characterised in that it is used as a stabilizing and gelling additive for foods.

8. Mitogen-free substance in accordance with one of Claims 1 to 3, characterised in that it is used as a wound closure for external and/or internal wounds and/or as a dental impression compound and/or, in the form of capsules or pellets, as a vehicle for pharmaceuticals or contrast media.

## Revendications

1. Substance non mitogène utilisant de l'acide guluronique ou de l'acide mannuronique, caractérisée par des copolymères constitués d'un pourcentage molaire de 10 à 90 % d'acide guluronique et du complément respectif à 100% d'acide mannuronique, et par un poids moléculaire des copolymères de 10 000 à 500 000 dalton.

2. Substance selon la revendication 1, caractérisée par sa biocompatibilté.

3. Substance non mitogène selon la revendication 1, caractérisée par une mobilité électrophorétique des copolymères de 3-5 . 10⁻⁴ cm² . V⁻¹ . s⁻¹.

4. Procédé de fabrication de la substance non mitogène selon l'une des revendications 1 à 3, caractérisé en ce qu'une solution jusqu'à 10% d'alginate est séparée en différentes fractions par électrophorèse libre, et ensuite on effectue une dialyse de la fraction possédant une mobilité électrophorétique de 3-5 . 10⁻⁴ cm² . V⁻¹ . s⁻¹, pour séparer les molécules ayant un poids de 10 000 à 500 000 dalton.

5. Procédé de fabrication d'une substance non mitogène selon l'une des revendications 1 à 3, caractérisé en ce que la solution d'alginate est précipitée en un complexe insoluble, par l'addition de barium ou de cations multivalents (par exemple plomb Pb, cuivre Cu), qui présentent une affinité comparable ou supérieure à celle du barium pour l'alginate, en ce que l'on effectue ensuite une extraction par l'acide, par exemple de l'acide acétique, durant plusieurs heures à température élevée, en ce qu'ensuite, après un lavage, est effectuée durant plusieurs heures, une autre extraction avec un agent complexant, par exemple de l'acide citrique, dans le domaine alcalin, en ce qu'ensuite, après un autre lavage avec de l'eau distillée, on effectue durant plusieurs heures, un traitement avec de l'alcool, par exemple de l'éthanol, et en ce que finalement on libère les alginates par addition d'E.D.T.A (acide éthylène diamine tétracétique ou acide édétique) dans le domaine fortement alcalin.

6. Substance non mitogène selon l'une des revendications 1 à 3, caractérisée en ce qu'elle est utilisée en tant qu'implant, de préférence en tant que capsules renfermant à l'intérieur des cellules vivantes.

7. Substance non mitogène selon l'une des revendications 1 à 3, caractérisée en ce qu'elle est utilisée en tant que stabilisant et gélifiant pour produits alimentaires.

8. Substance non mitogène selon l'une des revendications 1 à 3, caractérisée en ce qu'elle est utilisée en tant que pansement de lésions à usage interne et/ou externe, et/ou en tant que masse de prise d'empreinte en chirurgie dentaire, et/ou en tant que capsules, gélules ou billes servant de substance formant vecteur pour des médicaments ou des agents de contraste.
